# EUROPEAN PATENT SPECIFICATION

(11) **EP 2 116 471 B1**
(45) Date of publication and mention of the grant of the patent: **18.05.2016**
(21) Application number: 07849991.0
(22) Date of filing: 27.11.2007
(51) Int. Cl.: B65B 55/08, A61L 2/08, B65B 55/04, G21K 5/00, G21K 5/04

(54) **STERILITY KEEPING METHOD AND APPARATUS FOR STERILIZATION ELECTRON BEAM IRRADIATING DEVICE**
STERILHALTEVERFAHREN UND -VORRICHTUNG FÜR EINE STERILISATIONSELEKTRONENSTRAHLUNGSVORRICHTUNG
PROCÉDÉ ET APPAREIL DE MAINTIEN DE LA STÉRILITÉ POUR UN DISPOSITIF DE STÉRILISATION PAR IRRADIATION PAR FAISCEAU D'ÉLECTRONS

(30) Priority: 28.12.2006 JP 2006354258
(43) Date of publication of application: 11.11.2009
(73) Proprietor: Hitachi, Ltd., Tokyo (JP)
(72) Inventor: EGUCHI, Shiro, Tokyo 105-0003 (JP); GOHZAKI, Satoru, Ichihara-shi Chiba 290-0067 (JP); SUZUKI, Takayuki, Ichihara-shi Chiba 290-0067 (JP); HIKOSAKA, Tomoyuki, Ichihara-shi Chiba 290-0067 (JP); SATO, Shigekatsu, Hitachi-shi Ibaraki 316-8501 (JP); OKAMOTO, Yukio, Ichihara-shi Chiba 290-0067 (JP); HASHIMOTO, Isao, Hitachi-shi Ibaraki 319-1224 (JP)
(74) Representative: Strehl Schübel-Hopf & Partner
(86) International application number: PCT/JP2007/073288
(87) International publication number: WO 2008/081672

(56) References cited:
- EP-A1- 1 736 174
- WO-A2-2005/108278
- JP-A- H 111 212
- JP-A- 2002 171 949
- JP-A- 2002 171 949
- JP-A- 2003 054 521
- JP-A- 2003 054 521
- JP-A- 2003 180 799
- JP-A- 2006 314 407
- JP-A- 2006 314 407
- JP-A- 2007 297 067
- US-A1- 2005 158 218

## Description

### Technical Field

The present invention relates to a method for maintaining sterility and a sterilization electron beam irradiating apparatus for maintaining sterility, more particularly relates to a method for maintaining sterility and sterilization electron beam irradiating apparatus for maintaining sterility suitable for sterilization of irradiation targets such as containers or similar objects by electron beam irradiation.

### Background Art

Recent years, open-mouthed containers of plastic are widely used for packing beverage, food, medical supplies, and even cosmetics. The containers undergo a sterilization of their inside for germfree state before filling such items to be packed; thereafter they are filled with contents and then sealed. As an alternative to the sterilization of an open-mouthed container by chemical treatment, which requires a large-scaled facility, it has been proposed to sterilize inside and outside of such container by electron beam irradiation while transferring the container through an irradiation apparatus at a high-speed.

For example, JP10-268100A (Patent Literature 1) has proposed an electron beam irradiation apparatus. In that apparatus, an open-mouthed plastic container, such as a PET bottle, is transferred by a conveying means to the irradiation area, where an object in the area is irradiated with electron beam emitted from an electron beam generating means, with the center axis of the container intersecting at right angles with the transferring direction, namely, with the container laid flat on its side traversing the transferring direction. The container is further transferred by another conveying means having a slant bottom, which causes the container to roll down on that slant bottom. While rolling down, the container passes the irradiation area created by the electron beam generating means with its entire inside and outside faces efficiently sterilized by electron beam irradiation (refer to Patent Literature 1 for more details).

As another example, JP11-1212 (Patent Literature 2) has proposed a container sterilizer by means of electron beam. The sterilizer described in the literature has an electron beam generating means arranged vertical-longitudinally, on which an electron beam emission window is provided. The electron beam generating means and its predetermined neighboring area including the emission window is covered with a radiopaque material to form a sterilization treatment chamber. An open-mouthed container, the processing object to be sterilized, is transferred on a container conveying means to the entrance of the sterilization treatment chamber and conveyed out from its exit, with its position kept upright. The apparatus has a rotation means to cause the container on transfer to turn on its axis from the point immediately before the irradiation window until the container passes over the window. Thereby, the apparatus provides an enhanced efficiency of sterilization of the open-mouthed container yet offering reduced apparatus size.

Further, to enable sterilization of the inside of an open-mouthed container transferred in upright position by a conveying means into a sterilization treatment chamber, JP2002-104334 (Patent Literature 3) has proposed a sterilization method and apparatus of vessel. In the proposed art, a low energy electron beam emitted from an electron beam irradiation apparatus is deflected by alternating-current magnetic field to scan in the direction of container transfer and is shared by radially arranged nozzles so that containers will be irradiated one by one while traveling. This feature enables a single electron beam irradiation means to sterilize insides of plural containers with the overall size of the apparatus reduced.

In general, sterilization of beverage or food requires a line for handling such item to be kept in a clean and germfree state throughout entire handling lines from the conveying-in stage of sterilization-required object down to its conveying-out stage. As an example of measures for this requirement, there has been proposed an apparatus for sterilizing food in JP2003-245061 (Patent Literature 4). In this apparatus, the entirety of a food handling line including the irradiation treatment apparatus is enclosed to form a sterilizing compartment. Through this sterilizing compartment isolated from outside, a filtered germ-removed air is flown in the direction from the after-food-processing side to the before-food-processing side to prevent the food under processing on the handling line from floating-germ-adhering thereto.

In the electron beam irradiation apparatus defined in Patent Literature 1 cited above, the open-mouthed container is transferred in upright position, but is temporary laid flat on its side while traveling through the irradiation treatment zone. Therefore integrating this apparatus into a production line requires the apparatus to have a laying and raising mechanism. Such mechanism, when integrated however, largely lowers the line speed for transferring the open-mouthed container. Thus, the integrating of the apparatus is a hard problem for such a production line as is required to have high production efficiency in conveying open-mouthed containers.

In contrast, the container sterilizer, the electron beam irradiation apparatus described in Patent Literature 2, can be integrated into various production lines. In this application however, the production line should run at a reduced conveying speed or should use a high-energy electron beam generating means to sterilize fully both the inside and the outside of the open-mouthed container. This is because of the fact that, in this container sterilizer, each of the open-mouthed containers on being conveyed is irradiated sideways for sterilization by electron beam while it passes the electron beam irradiation window provided at only one location on the electron beam generating means in the apparatus.

In the sterilization apparatus described in Patent Literature 3, a single electron beam generating means should have a number of nozzles arranged radially to share emitted electron beam to irradiate each of open-mouthed containers on being conveyed consecutively on a production line. This requirement leads in a sophisticated apparatus structure. To sterilize fully both the inside and the outside of the open-mouthed container, the production line should run at a reduced conveying speed with a problem in the enhancement of the efficiency of the production line.

In such a case that Patent Literature 4 discloses, when the whole units in the apparatus including not only the electron irradiation treatment apparatus but also a packaging unit are to be enclosed to form a sterilizing compartment, the entirety of the sterilizing compartment becomes large and further a clean air supply system for flowing germ-removed air and an associated exhaust unit become larger in size. This problem prevents a production line from a use of an economical construction method. This kind of sterilization apparatus is able to prevent sterilized food from floating-fungi-adhering thereto by flowing clean air. However, if germs invade a portion inside the apparatus due to a certain reason while the apparatus is not in operation, such contaminated portion will be sterilized when the apparatus resumes the operation if the portion was in the area before irradiation treatment, but such contaminated portion will not be sterilized even after the resuming if such portion was in the area after sterilized. As an extreme development of this later case of happening, all foods after processing must be recalled; this is a very big problem once happens.

An object of the present invention is to provide a method for maintaining sterility for a sterilization electron beam irradiating apparatus, in which the method keeps the entire electron beam irradiation treatments, covering from the sending-in stage down to the sending-out stage of the irradiation target, in a germfree condition to a good state preventing the irradiation target from germ-adhering thereto.

Another object of the present invention is to provide a sterilization electron beam irradiating apparatus for maintaining sterility, in which the apparatus keeps the entire electron beam irradiation treatments, covering from the sending-in stage of the irradiation target down to the sending-out stage thereof, in a germfree condition to a good status with a simple structure.
JP 2003 054521 A discloses an apparatus with the features of the preamble of present claim 2.

### Disclosure of Invention

The method for maintaining sterility for sterilization electron beam irradiation apparatus by the present invention offered for solving the above-stated problems is: a method for maintaining sterility in an electron beam irradiation treatment of an irradiation target with an electron beam irradiation means conveying the irradiation target consecutively for irradiation using sterilization electron beam irradiation apparatus, the apparatus comprising: a pre-pressure adjusting bath; a post-pressure adjusting bath; an irradiation treatment bath having a pressure reducing means that maintains internal pressure thereof at negative state; and a rotary carrier arranged rotatively in each of the baths, wherein the pre-pressure adjusting bath and the post-pressure adjusting bath are integrally jointed to the side face portion of the irradiation treatment bath, the method is comprising the steps of: flowing clean air from the post-pressure adjusting bath side to the pre-pressure adjusting bath side and discharging the clean air from the pre-pressure adjusting bath; pressurizing the post-pressure adjusting bath at its adjoining side to the irradiation treatment bath to a pressure higher than the pressure at an air discharging port on the pre-pressure adjusting bath; and sterilizing gas existing in the joint between the irradiation treatment bath and the pre-pressure adjusting bath by irradiating the gas with electron beam.

The sterilization electron beam irradiating apparatus for maintaining sterility by the present invention also offered for is an apparatus comprising: a pre-pressure adjusting bath; a post-pressure adjusting bath; an irradiation treatment bath having a pressure reducing means that maintains internal pressure thereof at negative state, wherein the pre-pressure adjusting bath and the post-pressure adjusting bath are jointed to the side face portion of the irradiation treatment bath; a rotary carrier arranged rotatively in each of the baths that conveys consecutively an irradiation target; at least one electron beam irradiation means provided in the irradiation treatment bath to irradiate the irradiation target; an auxiliary electron beam generating means provided at the joint between the irradiation treatment bath and the pre-pressure adjusting bath for sterilizing gas by irradiation with electron beam; a clean air feeding means connected with each of the pre-pressure adjusting bath and the post-pressure adjusting bath severally through an air duct; and an exhaustive system connected to the inside of the pre-pressure adjusting bath.

Preferably, the sterilization electron beam irradiating apparatus for maintaining sterility by the present invention may be such that a germ migration control means is installed on a part of the conveying path in the irradiation treatment bath, in which path the rotary carrier moves from the post-pressure adjusting bath side toward the pre-pressure adjusting bath side.

### Effect of Invention

According to the method for maintaining sterility for sterilization electron beam irradiating apparatus of the present invention, clean air is fed with a pressure from the post-pressure adjusting bath located on the sending-out side of the irradiation target to the pre-pressure adjusting bath located on the sending-in side of the irradiation target and discharged there, and the internal pressure of the post-pressure adjusting bath adjacent to the irradiation treatment bath is made higher than the internal pressure of the pre-pressure adjusting bath adjacent to the irradiation treatment bath. Therefore, should the irradiation treatment process be invaded by germs adhered on the irradiation target, no germs will ambulate toward sending-out side of the irradiation target. Further, gas existing in the joint between the irradiation treatment bath and the pre-pressure adjusting bath is sterilized with reliability by electron beam irradiation. Thus, all the processing or treatment stages in a range from the sending-in of the irradiation target to the sending-out of the same after irradiation treatment are well kept germfree with largely enhanced reliability of the sterilization by electron beam irradiation.

Further, in the sterilization electron beam irradiating apparatus for maintaining sterility according to the present invention, the apparatus has such a feature: that at least one electron beam irradiation means is provided in the irradiation treatment bath to irradiate the irradiation target; that an auxiliary electron beam generating means is provided for sterilizing with electron beam the gas existing in the joint between the irradiation treatment bath and the pre-pressure adjusting bath; that a clean air feeding means is connected with each of the pre-pressure adjusting bath and the post-pressure adjusting bath severally through an air duct; and that an exhaustive system is connected to the inside of the pre-pressure adjusting bath. This simple structure, which permits the economical manufacturing of the whole apparatus, keeps all the processing or treatment stages in a range from the sending-in of the irradiation target to the sending-out the same in germfree.

### Brief Description of Drawings

Fig. 1 is a schematic illustration of a principle of the electron beam irradiation apparatus to which the present invention is applied.
Fig. 2 is a schematic plan view of an embodiment of the method for maintaining sterility and sterilization electron beam irradiating apparatus for maintaining sterility to which the present invention is applied.
Fig. 3 is a schematic vertical cross-section as viewed from the direction of line III-III in Fig. 2.
Fig. 4 is a pressure distribution chart in the method for maintaining sterility and sterilization electron beam irradiating apparatus for maintaining sterility defined in the present invention.

### Best Mode for Implementing the Invention

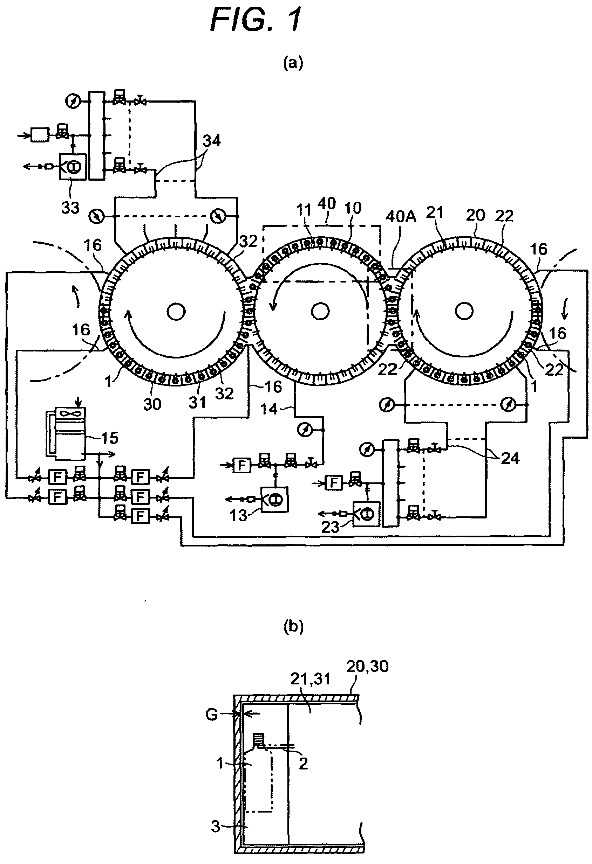

The principle of the electron beam irradiation apparatus to which the present invention is applied will be explained hereinafter referring to examples illustrated in Fig. 1 (a) and (b). In these examples, a plastic bottle is used as the irradiation target container.

A pre-pressure adjusting bath 20 and a post-pressure adjusting bath 30 are arranged adjacent to the side face of an irradiation treatment bath 10 located in the middle between the baths 20 and 30, which are integrally and severally jointed to the irradiation treatment bath 10. In the baths 10, 20, and 30, rotary carriers 11, 21, and 31, are rotatively arranged in order to severally form circular conveying paths between the walls of baths. The rotary carriers 11, 21, and 31, are rotated synchronously in the arrow-indicated directions by a driving mechanism, with which rotation a container 1 is transferred consecutively along the circular conveying paths. Thus, the processing line for the container 1 comes to have an arrangement of baths in the order of location: the pre-pressure adjusting bath 20, next the irradiation treatment bath 10, and lastly the post-pressure adjusting bath 30, wherein the pre-pressure adjusting bath 20 connects to a pre-treatment line and the post-pressure adjusting bath 30 connects to a post-treatment line.

Each of the rotary carriers 11, 21, and 31 has a plurality of retaining mechanisms 2 on its outer face at a regular interval to retain the container 1. This retaining mechanism 2 has such a structure as enables the smooth handing-over and receiving of the container 1 with the container's position kept upright while their traveling among the rotary carriers 21, 11, and 31 respectively arranged in the baths 20, 10, and 30 being located in the area from the pre-treatment line down to the post-treatment line.

The irradiation treatment bath 10 has a pressure tight sealed structure to endure the pressure reduction. A piping 14, which connects to a discharging means including an evacuation apparatus 13, is connected to the irradiation treatment bath 10; thereby the atmosphere around the container 1 on being transferred is maintained at a negative pressure of a predetermined level. Further, at least one electron beam generating means 40, which connects to a power supply, is provided on the irradiation treatment bath 10 at a portion thereof that works as the irradiation zone on the conveying path within the bath 10. Using the electron beam generating means 40, electron beam is emitted toward the portion of the conveying path, which portion works as the irradiation zone, in the irradiation treatment bath 10 maintained in a negative pressure to irradiate the container 1 being consecutively transferred for sterilization. In the present invention, the joint between the irradiation treatment bath 10 and the pre-pressure adjusting bath 20 is provided with an auxiliary electron beam generating means 40A to irradiate the gas in the joint with electron beam for sterilization. This auxiliary means contributes, jointly with a line configuration that will be mentioned later, to the maintaining of the entire line in a germfree state.

To each of the electron beam generating means 40 and the auxiliary means 40A, a device that works on lower acceleration voltages of 150 kV or lower is applicable; because the sterilization of the inside and the outside of the container 1 by irradiating with electron beam is performed in a negative pressure atmosphere, which does not require electron beam to have a high-energy. When the irradiation treatment bath 10 is in a pressure-reduced state, attenuation of the movement of beam is largely reduced. This means: that the electron range (range of flight) will be extended even if the energy of the electron is low; that the divergence will be small; and that therefore an effective irradiation of the inside of the container 1 is attainable even if the container has a narrow-mouth.

To enable to maintain the irradiation treatment bath 10 effectively at the negative pressure state facilitating a satisfactory irradiation, the present invention applies a particular arrangement to the pre-pressure adjusting bath 20, which connects to the pre-treatment line as the sending-in side of the container 1, and to the post-pressure adjusting bath 30, which connects to the post-treatment line as the sending-out side of the container 1. That is, the rotary carriers 21 and 31, respectively provided in the pre-pressure adjusting bath 20 and the post-pressure adjusting bath 30, are made have protrusions of partitions 3, each of which sections the adjacent retaining mechanisms 2 each from the other forming a plurality of small compartments 22 and 32 with the inside walls of the baths when the rotary carriers 21 and 31 rotate.

In the pre-pressure adjusting bath 20, to reduce the pressure in the small compartments 22 existing in the area between the point, where the container 1 is conveyed in from the preceding process line, and the other point, where the container 1 is transferred to the irradiation treatment bath 10, a plurality of piping 24 are connected on the bath wall of this area, wherein the piping 24 is connected to a discharging means including an evacuation apparatus 23. Thereby, the small compartments 22 in the area between the point, at which the container 1 is conveyed in from the preceding process line to the pre-pressure adjusting bath 20, and the other point, at which the container 1 is transferred to the irradiation treatment bath 10, are effectively controlled as a pressure regulated area of which pressure is adjusted within a specified range from atmospheric pressure down to a desired negative pressure.

In the post-pressure adjusting bath 30, to reduce the pressure in the small compartments 23 formed in the area between the irradiation treatment bath 10 and the point, at which the container 1 is conveyed out to the post-treatment line, a plurality of piping 34 are connected on the bath wall of this area similarly to the above-stated manner, wherein the piping 34 is connected to a discharging means including an evacuation apparatus 33. Thereby, the small compartments 32 in the area between the irradiation treatment bath 10 and the point, at which the container 1 is conveyed out to the post-treatment line, are effectively controlled as a pressure regulated area of which pressure is adjusted within a specified range from, in contrast, a desired negative pressure to atmospheric pressure.

The plural piping 24 and 34 are provided in such a manner that the pipe is made have a larger diameter, or instead, is made increase in piping number as the piping approaches the irradiation treatment bath 10, for making pressure-reduction efficient with a lightened capacity requirements for the evacuation apparatus 13, and for contributing a smooth pressure transition in the pre-pressure adjusting bath 20 and in the post-pressure adjusting bath 30. If necessary, the pressure reduction of the small compartment 32 in the pre-pressure adjusting bath 20 or in the post-pressure adjusting bath 30 can be applied to an area, if necessary, opposite to the above-stated area, by providing the discharging means thereon. More specifically, in the pre-pressure adjusting bath 20, the opposite area means an area in which the small compartments 32 move from the irradiation treatment bath 10 toward the preceding process line; and, in the post-pressure adjusting bath 30, the opposite area means an area in which the small compartments 32 move from the irradiation treatment bath 10 toward the succeeding process line.

Each of the partitions 3 for forming the small compartments 22 and 32 is arranged to create a narrow interstice G with the outer walls of the baths 20 and 30 as Fig. 1(b) illustrates. This construction means that one or more number of the partition 3 exist in the areas: between the irradiation treatment bath 10 and the open-to-atmosphere side of the pre-pressure adjusting bath 20, and between the irradiation treatment bath 10 and the open-to-atmosphere side of the post-pressure adjusting bath 30. Because of these arrangements, the plural partitions 3 works like a labyrinth seal increasing the flow resistance through the irradiation treatment bath 10 to the outside under atmospheric pressure. This increased flow resistance allows the irradiation treatment bath 10 to maintain its negative pressure state without particular sealing mechanism. Naturally, the capacity of the evacuation apparatus 13 to be provided on the irradiation treatment bath 10 as a discharging means should be determined allowing for the compensation of a possible leakage. Thereby, the predetermined negative pressure state of the irradiation treatment bath 10 is kept within a desired range.

Further to the above, a piping 16 led from a clean air generating device 15 with a suitably designed filter is connected to the irradiation treatment bath 10, to the pre-pressure adjusting bath 20, and to the post-pressure adjusting bath 30 to feed clean air to them using a dry-sealed pump. To provide such a gas atmosphere as permits a good irradiation of electron beam, a gas feeding device, which supplies various gasses such as nitrogen gas or helium gas, or their mixture, may be connected to the baths instead of the clean air generating device 15.

In the sterilization electron beam irradiating apparatus stated above, the container 1 conveyed out from the preceding process line in upright position and sent-in the pre-pressure adjusting bath 20 travels from, and via this order, the pre-pressure adjusting bath 20, the irradiation treatment bath 10, and the post-pressure adjusting bath 30, and then is sent-out therefrom to the successive process line. In the midway of this travel, the container 1 undergoes sterilization with electron beam irradiation emitted from the electron beam generating means 40 in the negative pressure atmosphere created in the irradiation treatment bath 10. In the area where the rotary carrier 21 in the pre-pressure adjusting bath 20 rotates clockwise under this situation, the small compartments 22, retaining the container 1 therein and existing in the area between the point, where the containers 1 are conveyed in from the preceding process line, and the other point, where the containers 1 approach the irradiation treatment bath 10, are pressure-reduced by a discharging means as they travel from the atmospheric pressure gradually to a negative pressure almost equal to the internal pressure of the irradiation treatment bath 10, when the compartments reach the rotary carrier 11 in the irradiation treatment bath 10.

In contrast, in the area where the rotary carrier 31 in the post-pressure adjusting bath 30 rotates clockwise, the small compartments 32, retaining the container 1 therein and existing in the area between the irradiation treatment bath 10 and the point where the containers 1 approaches the sent-out point to the succeeding process line, are brought gradually close to the atmospheric pressure from the negative pressure state in the irradiation treatment bath 10 by a discharging means with being returned to the atmospheric pressure when the compartments reach the point where the containers 1 are sent out to the succeeding process line.

The configuration of the sterilization electron beam irradiating apparatus defined as above enables the separate adjusting of the internal pressures of the irradiation treatment bath 10, the pre-pressure adjusting bath 20, and the post-pressure adjusting bath 30 to a desirable state; thereby the container 1 is sterilized effectively with electron beam in the irradiation treatment bath 10 maintained at a negative internal pressure using the electron beam generating means 40 at a lower energy rates. The sterilization electron beam irradiating apparatus has such a configuration that the irradiation treatment bath 10, the pre-pressure adjusting bath 20, and the post-pressure adjusting bath 30 are integrally jointed. The apparatus therefore provides such advantages further to the above that each of the open-mouthed containers are conveyed consecutively by the rotary carrier in each baths at a higher speed with being maintained upright position for irradiation in the particular area provided within the irradiation treatment bath 10 permitting in-line integration of the apparatus in a production line for such as beverages to work as a continuous sterilization unit of open-mouthed containers in the production line.

Furthermore, the apparatus transfers the containers 1 on the rotary carriers 11, 21, and 31 maintaining them in upright position, which imposes less force on elements in the transferring mechanism causing little wearing problems; therefore, the apparatus is a durable apparatus that works for a long time in a production line.

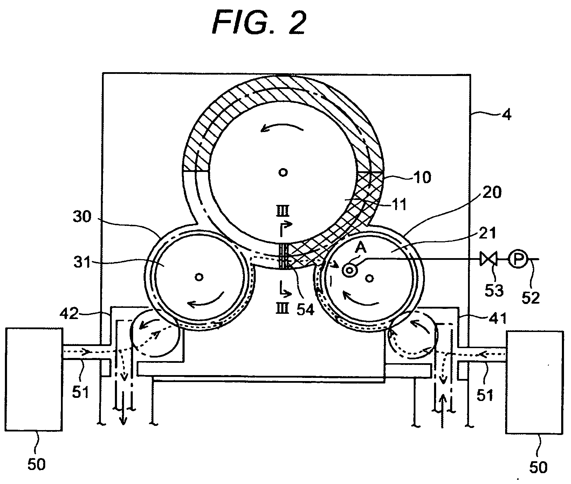

Fig. 2 shows an example of an approximate configuration of the sterilization electron beam irradiating apparatus according to the present invention. The diameter of the irradiation treatment bath 10 is designed to be larger than the diameters of the pre-pressure adjusting bath 20 and the post-pressure adjusting bath 30, to both of which the irradiation treatment bath 10 is integrally jointed; the entirety of these three baths are enclosed in a housing 4. The hatched area in the conveying path in the irradiation treatment bath 10 of Fig. 2 is such an area of the conveying path in which the irradiation target conveyed on the rotary carrier 11 is sterilized with irradiation of electron beam emitted from the electron beam generating means 40. Another hatched area in the joint between the irradiation treatment bath 10 and the pre-pressure adjusting bath 20 is such an area of the conveying path in which the gas existing the joint is sterilized with irradiation of electron beam emitted from the auxiliary electron beam generating means 40A provided at the joint.

The pre-pressure adjusting bath 20 is equipped with a sending-in chamber 41 for pretreatment of the irradiation targets conveyed from the preceding process line of linear layout in order that they should be transferred smoothly into the rotary carrier 21. The post-pressure adjusting bath 30 is equipped with a sending-out chamber 42 for post-treatment of the irradiation targets conveyed from the rotary carrier 31 in order that they should be transferred smoothly into the succeeding line of linear layout. Each of the circles of a dot-dash line shown in the figure severally indicates the traveling center of the retaining mechanism on the rotary carriers 11, 21, and 31, and the irradiation targets.

The irradiation treatment bath 10 is arranged on a supporting base structure (not shown) being integrally jointed with the pre-pressure adjusting bath 20 and the post-pressure adjusting bath 30. On the supporting base structure, a driving mechanism, such as a motor and a wheel, is arranged to synchronously drive the rotary carriers 11, 21, and 31 accommodated respectively in the baths 10, 20, and 30. The rotary carriers 11, 21, and 31 are arrayed in a narrow-width cable-reel-like style (or a carousel-like style), or installed on a hoop using supporting arms.

In the present invention, the sending-in chamber 41 installed on the pre-pressure adjusting bath 20 and the sending-out chamber 42 installed on the post-pressure adjusting bath 30 are severally connected to a clean air feeding means 50 through a blower duct 51; further, an exhaustive system 52 is connected to a discharge port A of the pre-pressure adjusting bath 20. Fig. 2 illustrates an example wherein the sending-in chamber 41 and the sending-out chamber 42 are severally connected with the clean air feeding means 50 provided individually therefor. Alternatively, it may be practicable to cover both the sending-in chamber 41 and the sending-out chamber 42 by one clean air feeding means 50 being given a large capacity linking the chambers with the blower duct 51.

The clean air feeding means 50 is equipped with a blower having sufficient capacity to feed germfree air through a filter or an air-cleaning treatment device. The discharge line 52, which connects into the pre-pressure adjusting bath 20, has a valve 53 and a discharge pump P on its piping system to permit regulating the amount of discharge from the pre-pressure adjusting bath 20 enabling controlling the internal pressure thereof at an intended level, which will be described later.

When a clean air is forcibly fed from the clean air feeding means 50 in the direction indicated with a broken-line arrow in this configuration, a part of the clean air fed therein diverts into the preceding process line and the succeeding process line preventing germs from invading the pre-pressure adjusting bath 20 and post-pressure adjusting bath 30, while the major part of the fed clean air goes into the pre-pressure adjusting bath 20 and the post-pressure adjusting bath 30 respectively through the sending-in chamber 41 and the sending-out chamber 42. In this air-flowing arrangement, the clean air fed into the post-pressure adjusting bath 30 travels therefrom to the pre-pressure adjusting bath 20 following mainly such a conveying path as is the shortest path to the pre-pressure adjusting bath 20, which path runs from the conveying path in the post-pressure adjusting bath 30 to the pre-pressure adjusting bath 20 via the returning portion of the conveying path in the irradiation treatment bath 10. The clean air is finally discharged through the discharge line 52 having a mechanism with which the amount of discharge is regulable. Thus, as the overall aspect, the flow of the clean air from the clean air feeding means 50 takes a path reverse to the traveling direction of the irradiation targets, that is, from the post-pressure adjusting bath 30 to the pre-pressure adjusting bath 20.

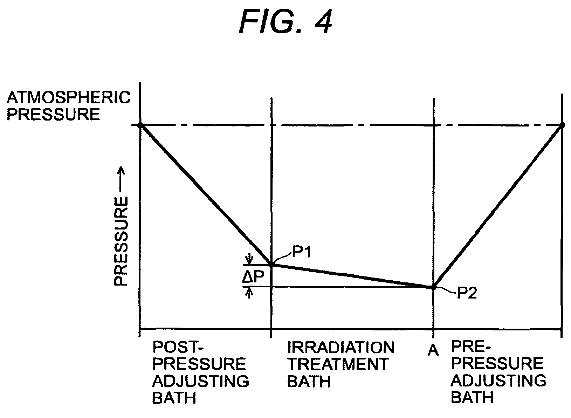

The pressure of the clean air fed from the clean air feeding measure 50 into the post-pressure adjusting bath 30 is the atmospheric pressure as Fig. 4 illustrates. The pressure of the clean air however gradually decreases as it flows because the internal pressure of the irradiation treatment bath 10 is maintained at a pressure-reduced state. The internal pressure of the post-pressure adjusting bath 30, particularly the pressure P1 at the area adjacent to the returning path portion of the irradiation treatment bath 10 where the rotary carriers move toward irradiation area thereof, is maintained at a pressure higher than the pressure P2 at the point of the discharge port A in the pre-pressure adjusting bath 20. Thereby, a pressure difference LP is always produced on the clean air stream between them.

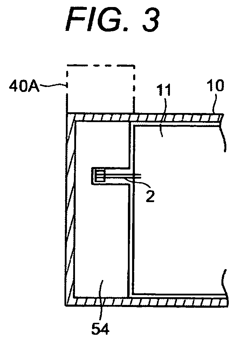

At the joint between the irradiation treatment bath 10 and the pre-pressure adjusting bath 20, the auxiliary electron beam generating means 40A is provided as illustrated in Fig. 3. With this arrangement, the auxiliary electron beam generating means 40A irradiates the gas existing in the joint with its electron beam for sterilization.

These configurations provide features: such that, should a germ-contaminated air invade the pre-pressure adjusting bath 20, such contaminated air is quickly discharged through the discharge line 52; such that the gas that passes the joint is sterilized by the electron beam emitted from the auxiliary electron beam generating means 40A; and such that an occurrence of germ invasion into the irradiation treatment bath 10 including successive downstream processes is eliminated since the clean air flows always from the post-pressure adjusting bath 30 toward irradiation treatment bath 10.

Germs, carried in as foreign adherents on the irradiation target and transferred and adhering to the rotary carrier 11 in the irradiation treatment bath 10, are sterilized by electron beam irradiation with reliability. Further, the area where germ may transfer from the pre-pressure adjusting bath 20 directly to the post-pressure adjusting bath 30 is swept by flow of the clean air. Moreover, the auxiliary electron beam generating means 40A is provided at the joint between the irradiation treatment bath 10 and the pre-pressure adjusting bath 20. These arrangements mean that the germ-prevention measure is enough.

To constrain the germ migration from the pre-pressure adjusting bath 20 to the post-pressure adjusting bath 30 while the electron beam irradiating apparatus is out of operation, a germ migration control means 54 is installed on a part of the returning portion of the conveying path in the irradiation treatment bath 10. The migration control means 54 has a structure such that, as Fig. 3 illustrates for example, a larger part of the returning conveying path in the irradiation treatment bath 10 where the retaining mechanism 2 on the rotary carrier 11 passes is closed with a rubber plate or a similar member leaving a clearance to permit the retaining mechanism 2 to pass. The auxiliary electron beam generating means 40A emits electron beam to irradiate the gas in the joint. Thereby, the area on the pre-pressure adjusting bath 20 side is kept in a sterility state.

The clean air feeding means 50 is connected to the pre-pressure adjusting bath 20 and the post-pressure adjusting bath 30, and the discharging line is connected to the pre-pressure adjusting bath 20. With this arrangement, the irradiation treatment bath 10 is kept pressure-reduced as stated above; thereby, even if there might be a possibility of occurrence of disturbance to a smooth flow of the clean air, pressure P2 at the discharging port A in the pre-pressure adjusting bath 20 is maintained at a proper level. This means that the apparatus is a self-contained system.

In the above-stated method for maintaining sterility and sterilization electron beam irradiating apparatus for maintaining sterility, the clean air is actively flowed in the direction reverse to the sending-out direction of the irradiation target. Therefore, there is no anxiety about the adhering of germs on the sterilized irradiation target that may be caused from the transferring of germ-contained air from the pre-pressure adjusting bath 20 toward the post-pressure adjusting bath 30. Accordingly, there will not occur recalling the irradiation targets after sterilization by electron beam, which feature is useful especially in the case where the irradiation targets are food-related items.

### Industrial Applicability

As stated above, the method for maintaining sterility and sterilization electron beam irradiating apparatus for maintaining sterility by the present invention is applicable to an electron beam irradiation treatment of an irradiation target with an electron beam irradiation means conveying the irradiation target consecutively for irradiation using sterilization electron beam irradiation apparatus, the apparatus comprising: a pre-pressure adjusting bath; a post-pressure adjusting bath; an irradiation treatment bath having a pressure reducing means that maintains internal pressure thereof at negative state; and a rotary carrier arranged rotatively in each of the baths, wherein the pre-pressure adjusting bath and the post-pressure adjusting bath are integrally jointed to the side face portion of the irradiation treatment bath. Further, the invented apparatus and method permit the entire processes involved in such apparatus arrangement covering from the sending-in stage of the irradiation target to the sending-out stage thereof to be kept in a good germfree condition with greatly enhanced reliability of the electron beam sterilization; and such apparatus and method are suitable to an economical construction of the entire arrangement.

## Claims

1. A method for maintaining sterility in an electron beam irradiation treatment of an irradiation target using a sterilization electron beam irradiation apparatus to which said irradiation target is conveyed consecutively for irradiation, the apparatus comprising:
a pre-pressure adjusting bath (20);
a post-pressure adjusting bath (30);
an irradiation treatment bath (10) having a pressure reducing means (13) that maintains internal pressure thereof at negative state; and
a rotary carrier (11, 21, 31) arranged rotatively in each of said baths (10, 20, 30), wherein said pre-pressure adjusting bath (20) and said post-pressure adjusting bath (30) are integrally jointed to the side face portion of said irradiation treatment bath (10),
an auxiliary electron beam generating means (40A) is provided at the joint between said irradiation treatment bath (10) and said pre-pressure adjusting bath (20) for sterilizing gas by irradiation with electron beam; and
a clean air feeding means (15) is connected with each of said pre-pressure adjusting bath (20) and said post-pressure adjusting bath (30) severally through an air duct (16).
said method comprising the steps of:
flowing clean air from said post-pressure adjusting bath side to said pre-pressure adjusting bath side and discharging said clean air from said pre-pressure adjusting bath (20);
pressurizing said post-pressure adjusting bath (30) at its adjoining side to said irradiation treatment bath (10) to a pressure higher than the pressure at an air discharging port on said pre-pressure adjusting bath (20); and
sterilizing gas existing in the joint between said irradiation treatment bath (10) and said pre-pressure adjusting bath (20) by irradiating said gas with said auxiliary electron beam.

2. A sterilization electron beam irradiating apparatus for maintaining sterility, the apparatus comprising:
a pre-pressure adjusting bath (20);
a post-pressure adjusting bath (30);
an irradiation treatment bath (10), wherein said pre-pressure adjusting bath (20) and said post-pressure adjusting bath (30) are jointed to the side face portion of said irradiation treatment bath (10);
at least one electron beam irradiation means provided in said irradiation treatment bath (10) to irradiate said irradiation target; and
an exhaustive system (23) connected to the inside of said pre-pressure adjusting bath (20),
**characterized in that**:
the irradiation treatment bath (10) has a pressure reducing means (13) that maintains internal pressure thereof at negative state;
a rotary carrier (11, 21, 31) is arranged rotatively in each of said baths (10, 20, 30) that conveys consecutively an irradiation target;
an auxiliary electron beam generating means (40A) is provided at the joint between said irradiation treatment bath (10) and said pre-pressure adjusting bath (20) for sterilizing gas by irradiation with electron beam; and
a clean air feeding means (15) is connected with each of said pre-pressure adjusting bath (20) and said post-pressure adjusting bath (30) severally through an air duct (16);

3. The sterilization electron beam irradiating apparatus for maintaining sterility according to claim 2, wherein a germ migration control means (54) is installed on a part of the conveying path in said irradiation treatment bath (10), in which path said rotary carrier (11, 21, 31) moves from said post-pressure adjusting bath side toward said pre-pressure adjusting bath side.

## Patentansprüche

1. Sterilhalte-Verfahren in einer Elektronenstrahl-Bestrahlungsbehandlung eines Bestrahlungsobjekts unter Verwendung eines Sterilisations-Elektronenstrahlbestrahlungsgeräts, zu dem das Bestrahlungsobjekt konsekutiv zur Bestrahlung befördert wird, wobei das Gerät aufweist:
ein Vordruck-Anpassungsbad (20),
ein Nachdruck-Anpassungsbad (30),
ein Bestrahlungsbehandlungsbad (10) mit einer Druckreduziereinrichtung (13), die deren Innendruck auf einem Unterdruck hält, und
einen Rotationsträger (11, 21, 31), der rotierbar in jedem der Bäder (10, 20, 30) angeordnet ist, wobei das Vordruck-Anpassungsbad (20) und das Nachdruck-Anpassungsbad (30) einstückig an dem Seitenflächenabschnitt des Bestrahlungsbehandlungsbads (10) angelenkt sind,
wobei eine Hilfselektronenstrahlerzeugungseinrichtung (40A) an der Verbindungsstelle zwischen dem Bestrahlungsbehandlungsbad (10) und dem Vordruck-Anpassungsbad (20) zum Sterilisieren von Gas durch Bestrahlung mit einem Elektronenstrahl vorgesehen ist, und
wobei eine Reinluftzuführeinrichtung (15) sowohl mit dem Vordruck-Anpassungsbad (20) als auch mit dem Nachdruck-Anpassungsbad (30) einzeln durch eine Luftleitung (16) verbunden ist,
wobei in dem Verfahren:
Reinluft von der Seite des Nachdruck-Anpassungsbads zu der Seite des Vordruck-Anpassungsbads geströmt wird und die Reinluft aus dem Vordruck-Anpassungsbad (20) ausgegeben wird,
das Nachdruck-Anpassungsbad (30) an seiner an das Bestrahlungsbehandlungsbad (10) angrenzenden Seite auf einen Druck gebracht wird, der höher ist als der Druck an einer Luftausgabeöffnung des Vordruck-Anpassungsbads (20), und
an der Verbindungsstelle zwischen dem Bestrahlungsbehandlungsbad (10) und dem Vordruck-Anpassungsbad (20) existierendes Gas durch Bestrahlung mit dem Hilfselektronenstrahl sterilisiert wird.

2. Sterilisations-Elektronenstrahlbestrahlungsgerät zum Sterilhalten, wobei das Gerät aufweist:
ein Vordruck-Anpassungsbad (20),
ein Nachdruck-Anpassungsbad (30),
ein Bestrahlungsbehandlungsbad (10), wobei das Vordruck-Anpassungsbad (20) und das Nachdruck-Anpassungsbad (30) an dem Seitenflächenabschnitt des Bestrahlungsbehandlungsbads (10) angelenkt sind,
wenigstens eine Elektronenstrahlbestrahlungseinrichtung in dem Bestrahlungsbehandlungsbad (10) zur Bestrahlung des Bestrahlungsobjekts vorgesehen ist, und
ein Ausstoßsystem (23) mit dem Inneren des Vordruck-Anpassungsbads (20) verbunden ist,
**dadurch gekennzeichnet, dass**
das Bestrahlungsbehandlungsbad (10) eine Druckreduziereinrichtung (13) aufweist, die deren Innendruck auf einem Unterdruck hält,
ein Rotationsträger (11, 21, 31) rotierbar in jedem der Bäder (10, 20, 30) angeordnet ist, um ein Bestrahlungsobjekt konsekutiv zu befördern,
eine Hilfselektronenstrahlerzeugungseinrichtung (40A) an der Verbindungsstelle zwischen dem Bestrahlungsbehandlungsbad (10) und dem VordruckAnpassungsbad (20) zum Sterilisieren von Gas durch Bestrahlung mit einem Elektronenstrahl vorgesehen ist, und
eine Reinluftzuführeinrichtung (15) sowohl mit dem Vordruck-Anpassungsbad (20) als auch mit dem Nachdruck-Anpassungsbad (30) einzeln durch eine Luftleitung (16) verbunden ist.

3. Sterilisations-Elektronenstrahlbestrahlungsgerät zum Sterilhalten nach Anspruch 2, wobei eine Keimmigrations-Steuereinrichtung (54) auf einem Teil des Förderpfads in dem Bestrahlungsbehandlungsbad (10) angebracht ist, wobei auf dem Pfad der Rotationsträger (11, 21, 31) von der Seite des Nachdruck-Behandlungsbads zu der Seite des Vordruck-Behandlungsbads bewegt wird.

## Revendications

1. Procédé pour maintenir la stérilité dans un traitement d'irradiation par faisceau d'électrons d'une cible d'irradiation en utilisant un appareil de stérilisation par irradiation par faisceau d'électrons vers lequel ladite cible d'irradiation est acheminée consécutivement en vue d'une irradiation, l'appareil comportant :
un bain de réglage pré-pression (20) ;
un bain de réglage post-pression (30) ;
un bain de traitement par irradiation (10) ayant un moyen de réduction de pression (13), qui maintient une pression interne de celui-ci à l'état négatif ; et
un transporteur rotatif (11, 21, 31) disposé de manière rotative dans chacun desdits bains (10, 20, 30), dans lequel ledit bain de réglage pré-pression (20) et ledit bain de réglage post-pression (30) sont intégralement reliés à la partie de face latérale dudit bain de traitement par irradiation (10),
un moyen de génération d'un faisceau d'électrons auxiliaire (40A) est agencé au niveau de la jonction entre ledit bain de traitement par irradiation (10) et ledit bain de réglage pré-pression (20) pour stériliser du gaz par irradiation à l'aide du faisceau d'électrons ; et
un moyen d'alimentation en air propre (15) est relié à chacun dudit bain de réglage pré-pression (20) et dudit bain de réglage post-pression (30) séparément à travers une conduite d'air (16) ;
ledit procédé comprenant les étapes consistant à :
faire circuler de l'air propre à partir dudit côté du bain de réglage post-pression vers ledit côté du bain de réglage pré-pression et évacuer ledit air propre dudit bain de réglage pré-pression (20),
mettre sous pression ledit bain de réglage post-pression (30) au niveau de son côté voisin dudit bain de traitement par irradiation (10) à une pression supérieure à la pression au niveau d'un orifice d'évacuation de l'air sur ledit bain de réglage pré-pression (20), et
stériliser le gaz qui se trouve à la jonction entre ledit bain de traitement par irradiation (10) et ledit bain de réglage pré-pression (20) en irradiant ledit gaz avec ledit faisceau d'électrons auxiliaire.

2. Appareil de stérilisation par irradiation par faisceau d'électrons pour maintenir la stérilité, l'appareil comportant :
un bain de réglage pré-pression (20) ;
un bain de réglage post-pression (30) ;
un bain de traitement par irradiation (10), dans lequel ledit bain de réglage pré-pression (20) et ledit bain de réglage post-pression (30) sont reliés à la partie de face latérale dudit bain de traitement par irradiation (10) ;
au moins un moyen d'irradiation par faisceau d'électrons agencé dans ledit bain de traitement par irradiation (10) pour irradier ladite cible d'irradiation ; et
un système complet (23) relié à l'intérieur dudit bain de réglage pré-pression (20),
**caractérisé en ce que**
le bain de traitement par irradiation (10) dispose d'un moyen de réduction de pression (13) qui maintient une pression interne de celui-ci à l'état négatif ;
un transporteur rotatif (11, 21, 31) est disposé de manière rotative dans chacun desdits bains (10, 20, 30), qui achemine consécutivement une cible d'irradiation ;
un moyen de génération de faisceau d'électrons auxiliaire (40A) est agencé au niveau de la jonction entre ledit bain de traitement par irradiation (10) et ledit bain de réglage pré-pression (20) pour stériliser du gaz par irradiation avec le faisceau d'électrons ; et
un moyen d'alimentation en air propre (15) est relié à chacun dudit bain de réglage pré-pression (20) et dudit bain de réglage post-pression (30) séparément à travers une conduite d'air (16).

3. L'appareil de stérilisation par irradiation par faisceau d'électrons pour maintenir une stérilité selon la revendication 2, dans lequel un moyen de commande de migration de germes (54) est installé sur une partie du trajet d'acheminement dans ledit bain de traitement par irradiation (10), ledit transporteur rotatif (11, 2.1, 31) se déplaçant sur ce trajet depuis ledit côté du bain de réglage post-pression vers ledit côté du bain de réglage pré-pression.
